# EUROPÄISCHE PATENTSCHRIFT

(11) **EP 0 811 694 B1**
(45) Veröffentlichungstag und Bekanntmachung des Hinweises auf die Patenterteilung: **12.03.2003**
(21) Anmeldenummer: 97109177.2
(22) Anmeldetag: 06.06.1997
(51) Int. Cl.: C12Q 1/68, G01N 33/546, G01N 33/552

(54) **Reagenzzubereitung enthaltend magnetische Partikel in Form einer Tablette**
Reagent-composition with magnetic particles in the form of a tablet
Composition de réactifs avec des particules magnétiques en forme de comprimé

(30) Priorität: 07.06.1996 DE 19622885
(43) Veröffentlichungstag der Anmeldung: 10.12.1997
(73) Patentinhaber: Roche Diagnostics GmbH, 68298 Mannheim (DE)
(72) Erfinder: Harttig, Herbert, Dr., 67122 Altrip (DE)

(56) Entgegenhaltungen:
- WO-A-90/06045
- US-A- 3 981 776
- US-A- 4 115 534
- US-A- 4 169 804
- MARKO M A ET AL: "A PROCEDURE FOR THE LARGE-SCALE ISOLATION OF HIGHLY PURIFIED PLASMID DNA USING ALKALINE EXTRACTION AND BINDING TO GLASS POWDER" ANALYTICAL BIOCHEMISTRY, Bd. 121, Nr. 2, April 1982, Seiten 382-387, XP000602405
- FUJIMORI ET AL.: "Preparation ofa magnetically-resposive tablet and confirmation of its gastric residene in beagle dogs" S.T.P. PHARMA SCIENCES, Bd. 4, Nr. 6, 1994, Seiten 425-430, XP002084676
- VOGELSTEIN B ET AL: "PREPARATIVE AND ANALYTICAL PURIFICATION OF DNA FROM AGAROSE" PROCEEDINGS OF THE NATIONAL ACADEMY OF SCIENCES OF THE USA, Bd. 76, Nr. 2, Februar 1979, Seiten 615-619, XP000609019

## Beschreibung

Gegenstand der Erfindung ist eine Reagenzzubereitung zur Bindung von Inhaltsstoffen einer Probe in Form einer Tablette, deren Verwendung zur Bindung oder Reinigung von Nukleinsäuren sowie ein Verfahren zur Suspension von magnetischen Partikeln in einer Probe und ein Verfahren zum Einbringen von magnetischen Partikeln in einer Probe.

Bei der Analytik von flüssigen Proben stellt sich oft das Problem, daß die zu analysierenden Bestandteile nur in sehr geringen Mengen vorhanden sind. Darüber hinaus befinden sich in der Probe eine Vielzahl nicht zu bestimmender Bestandteile, die die Bestimmungen ungenau werden lassen. Aus diesem Grund hat es sich als zweckmäßig erwiesen, die Analyten an eine feste Phase zu binden und die nicht zu bestimmenden Bestandteile mit der Flüssigkeit zu entfernen. Die isolierten Analyten können anschließend an der festen Phase nachgewiesen werden. Als feste Oberflächen wurden in jüngerer Zeit insbesondere die Innenwände von Reaktionsgefäßen (Tubes) eingesetzt. Eine weitere Möglichkeit besteht darin, der Probe in einem Reaktionsgefäß eine Kugel (Bead) zuzusetzen, die für die Bindung des Analyten entsprechend befähigt ist. Diese Perlen sind so groß, daß die Abtrennung der Flüssigkeiten von diesen Perlen durch einfache Pipettierung möglich ist. In jüngerer Zeit wurden nun jedoch auch kontinuierlich arbeitende Geräte entworfen, bei denen der Analyt an magnetische Partikel gebunden wird und der gebundene Analyt zusammen mit dem magnetischen Partikel über ein Magnetfeld von der sie umgebenden Flüssigkeit abgetrennt werden. Auch hier sind die magnetischen Partikel mit einer Oberfläche versehen, die die Bindung des Analyten erlaubt.

Diese magnetpartikelhaltigen Reagenzzubereitungen werden in Form von Suspensionen angeboten, welche der zu untersuchenden analythaltigen Flüssigkeit zupipettiert werden kann. Diese Pipettierschritte sind den üblichen Schwankungen bei Pipettierungen unterworfen. Darüber hinaus sind Fehler bei den Pipettierungen schwer erkennbar.

US 4,115,534 offenbart die Bestimmung der Konzentration verschiedener Substanzen wie z.B. Arzneimittel, Hormone, Vitamine und Enzyme wobei magnetische, permeable, feste, wasserunlösliche Mikropartikel verwendet werden.

US 4,169,804 offenbart magnetische Mikropartikel, die bei der Konzentrationsbestimmung von Substanzen in biologischen Flüssigkeiten verwendet werden können. Die Offenbarung von US 4,169,804 ist ähnlich zu der Offenbarung von US 4,115,534. US 4,169,804 offenbart ferner, dass die Mikropartikel zu Tabletten geformt werden können.

US 3,981,776 offenbart biologisches Material, das mit magnetischem Material zu einem Formkörper geformt wird, um Diffusionsversuche auszuführen, die im Rahmen mikrobiologischer, immunologischer, serologischer oder biochemischer Untersuchungen durchgerührt werden. Der Formkörper wird durch Anlegen eines magnetischen Feldes auf ein Substrat oder Medium aufgebracht und eine Reaktionsregion wird produziert, die an einer Seite des Formkörpers ausgelesen wird. Der Formkörper kann auch durch einen Dispenser appliziert werden.

DE 195 20 398 offenbart magnetische Partikel mit einer äußeren Glasoberfläche, die im wesentlichen porenfrei ist oder Poren eines Durchmessers von weniger als 10 nm aufweist. Die Partikel sind bevorzugt einsetzbar bei der Isolierung von biologischen Materialien aus Proben. Sie ermöglichen eine rasche und zuverlässige Reinigung insbesondere von Nukleinsäuren. DE 195 20 398 offenbart auch die Herstellung der offenbarten magnetischen Partikel.

Aufgabe der vorliegenden Erfindung war es daher, die Nachteile des Standes der Technik zu beseitigen und insbesondere Magnetpartikel in einer Form anzubieten, die eine einfache Dosierbarkeit erlaubt.

Gegenstand der Erfindung ist daher eine Reagenzzubereitung zur Bindung von Inhaltsstoffen einer Probe in Form einer Tablette enthaltend eine Vielzahl von magnetischen Partikeln mit einer Oberfläche, an welche die zu bindenden Inhaltsstoffe im wesentlichen vollständig binden können und Tablettierhilfsmittel. Ebenfalls Gegenstand der Erfindung sind Verwendungen dieser Reagenzzubereitungen und Verfahren zur Herstellung von magnetischen Suspensionen.

Unter Inhaltsstoffen einer Probe werden Materialien auf partikulärer oder molekularer Basis verstanden. Hierzu gehören insbesondere Zellen, z. B. Viren oder Baktieren, aber auch humane oder tierische isolierte Zellen, wie Leukozyten, sowie immunologisch aktive niederund hochmolekulare chemische Verbindungen, wie Haptene, Antigene, Antikörper und Nukleinsäuren. Besonders bevorzugt als Inhaltsstoffe sind Nukleinsäuren, z. B. DNA oder RNA.

Proben im Sinne der Erfindung sind beispielsweise klinische Proben, wie Blut, Serum, Mundspülflüssigkeit, Urin, Zerebralflüssigkeit, Sputum, Stuhl, Punktate und Knochenmarkproben. Die Probe kann auch aus dem Bereich der Umweltanalytik, der Lebensmittelanalytik oder der molekularbiologischen Forschung, z. B. aus Bakterienkulturen, Phagenlysaten und Produkten von Amplifikationsverfahren, z. B. PCR, stammen.

Eine Tablette im Sinne der Erfindung ist ein fester Formkörper, bevorzugt in Form einer flachen Scheibe oder in Form einer mehr oder weniger perfekten Kugel. Dazwischenliegende Ausführungsformen sind ebenfalls denkbar. Solche Tabletten sind in ihrer Form beispielsweise aus üblichen Arzneimittelzubereitungen bekannt. Bevorzugt hat die Tablette ein im voraus definiertes Gewicht, welches schwerer ist als 5 mg.

Unter einem magnetischen Partikel werden Partikel aus Materialien bezeichnet, die durch einen Magnet angezogen werden können, d. h. beispielsweise ferromagnetische oder superparamagnetische Materialien. Besonders bevorzugt im Sinne der Erfindung sind ferromagnetische Materialien, insbesondere wenn sie noch nicht vormagnetisiert wurden. Unter Vormagnetisierung ist in diesem Zusammenhang das Inkontaktbringen mit einem Magneten zu verstehen, wodurch die Remanenz erhöht wird. Besonders bevorzugt sind Magnetid (Fe₃O₄) oder Fe₂O₃. Unter magnetischen Partikeln sollen jedoch auch Materialien verstanden werden, welche selbst (kleinere) magnetische Partikel enthalten. Hierzu gehört insbesondere das unter der Bezeichnung Iriodin 600 (Merck, Darmstadt) käuflich erhältliche Pigment. Besonders bevorzugt im Sinne der vorliegenden Erfindung sind Partikel, die eine durchschnittliche Korngröße von weniger als 100 µm haben. Besonders bevorzugt weisen sie eine durchschnittliche Korngröße von zwischen 10 und 60 µm auf. Bevorzugt ist die Korngrößenverteilung relativ homogen, insbesondere liegen nahezu keine Teilchen kleiner als 10 µm oder größer als 60 µm vor. Partikel, die diese Bedingung erfüllen, sind beispielsweise beschrieben in WO 90/06045.

Ein wesentlicher Bestandteil der Erfindung ist die Tatsache, daß die magnetischen Partikel eine Oberfläche aufweisen, an welche die zu bindenden Inhaltsstoffe binden können. Diese Bindung kann entweder spezifisch oder relativ unspezifisch sein. Eine spezifische Bindung kann beispielsweise dadurch erreicht werden, daß für die Bindung spezifische Wechselwirkungen, wie z. B. zwischen Antikörpern und Antigenen, Antikörpern und Haptenen oder komplementären Nukleinsäuren verwendet werden. Auch die Kombination solcher Wechselwirkungen ist möglich.

Eine bekannte Möglichkeit der Modifizierung einer Oberfläche ist beispielsweise der Überzug von Partikeln mit einer Schicht aus Streptavidin. Hierdurch läß sich eine universell verwendbare Matrix erzeugen, an die über Konjugate von Biotin mit einem bestimmten Antikörper, einem bestimmten Hapten oder einer bestimmten Nukleinsäure spezifische Inhaltsstoffe aus der Probe gebunden werden können. Ausführungsformen sind dem Fachmann, insbesondere auf dem Gebiet der Immunoassays, bekannt.

Unter einer relativ unspezifischen Bindung wird auch die Wechselwirkung zwischen einer glasartigen Oberfläche und Nukleinsäuren verstanden. So ist beispielsweise aus Proc. Natl. Acad. USA 76, 615 - 619 (1979) die Bindung von Nukleinsäuren aus Agarosegelen in Gegenwart von Natriumjodid in gemahlenem Flintglas bekannt. Magnetische Partikel, an deren Glasanteil Nukleinsäuren binden können, sind beispielsweise aus US-A-4,233,169 bekannt.

Im Sinne der Erfindung soll der zu bestimmende Inhaltsstoff im wesentlichen vollständig an die magnetischen Partikel binden können. Die Menge an hierfür erforderlichen magnetischen Partikeln kann ein Fachmann durch Variation der Menge an zugegebenen magnetischen Partikeln sehr leicht herausfinden. Im Sinne der Erfindung bedeutet eine im wesentlichen vollständige Bindung eine Bindung von mehr als 60 %, besonders bevorzugt mehr als 90 % der Probemenge des zu bindenden Inhaltsstoffes.

Tablettierhilfsmittel dienen im wesentlichen dazu, die feste Form der Tablette zu erhalten, d. h. die magnetischen Partikel miteinander zu einer Tablette zu vereinigen. Bevorzugte Tablettierhilfsmittel sind im Sinne der Erfindung solche, die sich in der Probe, in der die Bindung stattfinden soll, schnell lösen können. Da es sich bei den flüssigen Proben bevorzugt um wässrige Lösungen handelt, können praktisch alle auch für Arzneimittel eingesetzten Tablettierhilfsmittel verwendet werden. Als besonders bevorzugt haben sich Polyethylenglykol (PEG) und Polyvinylpyrrolidon (PVP) erwiesen.

In DE-A-4406139 ist ein magnetisches Depotarzneimittel mit verbesserter Resorption der Wirkstoffe beschrieben. Die Tablette enthält einen scheibenförmigen Magnet und die Tablette setzt den Wirkstoff über mehrere Stunden hinweg Frei.

In International Journal of Pharmaceutics 119, 47 - 55 (1995) ist ebenfalls eine Tablette beschrieben, mit der eine verzögerte Freisetzung von Arzneimitteln erreicht wird.

In STP Pharmasciences Vol. 4, 425 - 430 (1994) ist die Herstellung ferrithaltiger magnetischer Tabletten und deren Verabreichung an Hunde beschrieben.

Die erfindungsgemäße Tablette kann darüber hinaus auch noch Stabilisierungsreagenzien enthalten. Hierbei hat es sich als bevorzugt erwiesen, Zucker zuzusetzen, z. B. D-Mannit, Trehalose oder Sorbit.

Erstaunlicherweise lassen sich magnetische Partikel, insbesondere solche mit einer Glasoberfläche, in Form einer Tablette ohne beobachtbare Hydrolyse des Glases und damit auch ohne beobachtbare Elution des Eisens aus den Magnetanteilen, lagern.

Bei den magnetischen Partikeln handelt es sich bevorzugt um Glasmagnetpigmente oder . polymere Magnetbeads oder andere magnetische Partikel im Größenbereich von 0.1 µm bis 100 µm.

Der Zubereitung können weiterhin Hilfsstoffe zugesetzt sein, die die Bindung der Inhaltsstoffe erleichtern. Hierzu gehören beispielsweise spezifitätsvermittelnde Stoffe, wie die oben erwähnten Konjugate, aber auch Stoffe, die die Eigenschaften der Probe dahingehend verändern, daß die zu bindenden Inhaltsstoffe leichter an die Oberfläche binden. Im Falle von Nukleinsäuren sind dies beispielsweise chaotrope Salze, wie z. B. Guanidiniumhydrochlorid, Natriumjodid, Natriumperchlorat oder ähnliche. Solche chaotropen Salze sind z. B. aus Anal. Biochem. 121, 382 - 387 (1982) und DE-A 37 34 442 bekannt.

Die Reagenzzubereitung kann außerdem noch Reagenzien enthalten, die die zu bindenden Inhaltsstoffe in eine Form überführen, sodaß sie überhaupt für eine Bindung zugänglich ist. Hierzu gehören beispielsweise Reagenzien, mit denen Kompartimente, z. B. Zellen, die Nukleinsäuren enthalten, lysiert werden können. Ein solches Reagenz ist beispielsweise Proteinase K oder die oben erwähnten chaotropen Salze.

Die Reagenzzubereitung kann auch pH-Puffersubstanzen und Reagenzien zur Lösung von Wechselwirkungen, wie z. B. Wasserstoffbrückenbindungen, Hydrophobenwechselwirkungen, ionischen Bindungen sowie Reagenzien zum spezifischen Nachweis von Substanzen oder Indikatoren, wie sie an sich als Komponenten eines immunologischen Assays bekannt sind, enthalten.

Für eine bevorzugte Tablette hat sich folgende Zusammensetzung als praktikabel erwiesen:

| **Bestandteil** | **bevorzugte** **Menge** | **besonders bevorzugte** **Menge** |
|---|---|---|
| Tablettierhilfsmittel (z.B. PEG, PVP, Kalziumstearat) | 2 - 10 % | 3 % |
| Reagenzien | 0 - 90 % | 87 % |
| Magnetische Partikel | 0.01 - 50 % | 10 % |

Selbstverständlich kann eine erfindungsgemäße Tablette weitere Bestandteile beinhalten, z. B. inerte Füllstoffe; die Gesamtmenge an Bestandteilen addiert sich zu 100 %. Die angegebenen Prozentzahlen sind Gewichtsprozent.

Die erfindungsgemäße Reagenzzubereitung in Form einer Tablette kann in Analogie zu Arzneimitteln in Tablettenform hergestellt werden. Hierzu werden die erforderlichen Komponenten miteinander innig vermischt und Aliquots in einer Tablettenpresse tablettiert. Dies geschieht insbesondere durch Einwirkung von Druck. Erfindungsgemäße Tabletten können jedoch auch durch Granulieren der Mischung der Komponenten erzeugt werden. Hierzu wird eine bestimmte Menge an trockener Mischung mit einer lösungsvermittelnden Flüssigkeit granuliert. Dem erhaltenen Granulat wird die Flüssigkeit anschließend wieder entzogen. Eine gleichmäßige Korngröße kann durch Sieben des Granulats erreicht werden.

Diese Herstellungsverfahren bewirken einen sehr niedrigen Variationskoeffizienten des Tablettengewichts und damit auch eine hohe Reproduzierbarkeit bei der Dosierung der Reagenzien im Anwendungsfall. Fehler bei der Dosierung können weniger oft stattfinden bzw. werden schneller bemerkt. Die erfindungsgemäßen Tabletten lassen sich sehr rasch auflösen, bevorzugt in weniger als 30 sec., besonders bevorzugt in zwischen 1 bis 10 sec., und die Magnetpartikel lassen sich einfach und schnell redispergieren. Die Tablettenform ist eine gut lagerfähige Form. Die Dosierung kann beispielsweise sogar manuell mit Hilfe eines Tablettenspenders erfolgen. Die bei Suspensionen auftretenden Verfälschungen durch Sedimentierung der Partikel sind nicht zu beobachten.

Ebenfalls Gegenstand der Erfindung ist die Verwendung der Reagenzzubereitung zur Bindung von Nukleinsäuren. Hierzu wird die Reagenzzubereitung der Probe zugegeben und solange inkubiert, bis sich 1.) die Tablette aufgelöst und 2.) die Nukleinsäuren im wesentlichen vollständig an die Oberfläche gebunden sind. Hierzu kann die Tablette mechanisch bewegt werden. Dies erhöht sowohl die Geschwindigkeit bei der Auflösung der Tablette als auch die Geschwindigkeit bei der Bindung der Inhaltsstoffe.

Ebenfalls Gegenstand der Erfindung ist die Verwendung der Reagenzzubereitung zur Reinigung von Nukleinsäuren. Hierzu werden die magnetischen Partikel mit den daran gebundenen Nukleinsäuren von der sie umgebenden Probenflüssigkeit entfernt. Dies geschieht zweckmäßigerweise durch Anlegen eines Magnetfeldes zum Zurückhalten der Magnetpartikel in einem Gefäß oder an einer bestimmten Stelle eines Apparates und anschließende Entfernung (z. B. durch Abpipettierung oder Verdrängung) der Probenflüssigkeit sowie gewünschtenfalls einer oder mehrerer Waschschritte mit anderen Flüssigkeiten. Falls gewünscht können die gebundenen Nukleinsäuren durch Anlegung geeigneter Bedingungen wieder von den magnetischen Partikeln gelöst werden. Im Falle einer glasartigen Oberfläche sind diese bevorzugt Niedrigsalzbedingungen, d. h. der Salzgehalt der Elutionslösung liegt bei weniger als 100 mmol/l.

Ebenfalls Gegenstand der Erfindung ist ein Verfahren zur Suspension von magnetischen Partikeln in einer Probe, enthaltend die Schritte Zugabe einer magnetische Partikel und lösliche Tablettierhilfsstoffe enthaltenden Tablette in die Probe und Bewegung der Tablette in der Probe, bevorzugt mit Hilfe eines beweglichen Magnetfeldes. Die Bewegung des Magnetfeldes kann z. B. dadurch erreicht werden, daß ein Magnet in der Nähe der Probe hin- und herbewegt wird, so daß die Tablette bzw. die magnetischen Partikel eine ständige Ortsveränderung mitmachen. Es ist jedoch auch möglich, das Gefäß, in welchem sich die Probe mit der Tablette bzw. den magnetischen Partikeln befindet, gegenüber dem Magneten zu bewegen.

Ebenfalls Gegenstand der Erfindung ist ein Verfahren zum Einbringen von magnetischen Partikeln in eine Probe enthaltend die Schritte zur Verfügungstellung eines Spenders enthaltend eine Vielzahl von magnetpartikelhaltigen Tabletten und Betätigung des Spenders zum Entlassen einer Tablette aus dem Spender. Spender für die Zurverfügungstellung von Tabletten sind beispielsweise bei der Verabreichung von Medikamenten in Tablettenform gebräuchlich. Diese können beispielsweise in manueller Weise zur Dosierung im erfindungsgemäßen Verfahren eingesetzt werden. Es ist hier nicht unbedingt erforderlich, daß nur eine Tablette pro Probe freigesetzt wird. Es ist auch möglich, eine bestimmte Anzahl von Tabletten, z. B. zwischen 2 und 10 Tabletten, abhängig von der beabsichtigten Verwendung in der Probe, zu entlassen.

Die folgenden Beispiele sollen die Erfindung näher erläutern:

### Beispiel 1

### Herstellung des Glasmagnetpigments

Die Herstellung eines Sols (SiO₂:B₂O₃ = 7:3) wurde in einem 250 ml Rundkolben unter ständigem Rühren nach folgender Vorschrift durchgeführt:
86,6 ml Tetraethylorthosilicat
+ 7 ml wasserfreies unvergälltes Ethanol
+ 14,1 ml 0,15 M HCl

Es entsteht ein zweiphasiges Gemisch, welches bei Raumtemperatur solange gerührt wird, bis es einphasig wird. Danach erfolgt tropfenweise die Zugabe von 37,8 ml Trimethylborat. Anschließend wird das Sol 2 Stunden bei 50 °C gehalten. Danach erfolgt die Zugabe von 14,1 ml 0,15 M HCl

Nach Alterung wurde in 150 ml des Sols 22,5 g Iriodin 600 (Black Mica, Merck, Darmstadt, BRD) eingerührt und anschließend mit einem Sprühtrockner (Büchi 190, Mini Spray Dryer) beschichtet.

Das durch den Sprühtrockenprozeß erhaltene Pulver wurde anschließend einer Temperaturbehandlung unter Stickstoffatmosphäre unterzogen. Die Aufheizgeschwindigkeit betrug hierbei 1 K/min und die Haltezeit betrug 2 Stunden bei der Verdichtungstemperatur. Nach dem Verdichtungsprozeß wurde die Temperatur auf die Nachbehandlungstemperatur abgesenkt, die Stickstoffatmosphäre durch Luft ersetzt und das Pulver nach Ablauf der Nachbehandlung auf Raumtemperatur abgekühlt. Eventuell entstandene Agglomerate wurden mit einem 50 µm Sieb ausgesiebt.

| **Parameter** | **GMP 2** |
|---|---|
| Alterung des Sols bei 30°C (h) | 36 |
| Pigmentanteil des Sols (g/100 ml) | 15 |
| Düsentemperatur (°C) | 120 |
| Luftstrom der Düse (%) | 100 |
| Luftdruck (bar) | 6 |
| Verdichtungstemperatur (°C) | 534 |
| O₂-Nachbehandlung (1 Stunde) | (300°C) |

### Beispiel 2

### Herstellung der Tablette

### Vormischung

43,62 g Glasmagnetpigment GMP 2 wurden mit 500 g Guanidiniumhydrochlorid gemischt und durch ein 0,2 mm Sieb auf einer Frewiit-Siebmaschine, Typ GLA-ORV, gesiebt. Die Ausbeute betrug 536,4 g, dies enspricht 98,7 %.

### Granulieren

0,674 g Tris-HCI und 0,259 g Harnstoff wurden in 2,2 ml bidest. Wasser gelöst. Die Lösung wurde zusammen mit 266,4 g der Vormischung granuliert. Dabei wurden insgesamt 7 ml H₂O-bidest. zugegeben. Das erhaltene Granulat wurde im Vakuum bei Raumtemperatur über 24 Std. getrocknet und anschließend durch ein 0,6 mm Sieb gesiebt.

### Tablettieren

246,98 g des Granulats wurden mit 7,41 g PEG 6000, sprüherstarrt, gemischt und auf einer Korsch PH106 Tablettenpresse mit einer Stempelgröße von 5 mm tablettiert. Die Ausbeute betrug 186,63 oder 2902 Tabletten. Die Tabletten hatten ein Gewicht von 64,32 mg, eine Tablettenhärte von 14,71 N (1,5 kp), eine Auflösezeit in dest. Wasser bei Raumtemperatur von 6 sec., einen Abrieb von 0,8 % und eine Tablettenhöhe von 2,77 mm.

Die Tablettiermaschine und Stempel ließen sich gut reinigen, die Matrizen blieben blank, die Stempel waren nicht belegt. Die Masse lief etwas stockend, dies äußerte sich in einem Gewichts-VK von 4,65 % und kann durch technische Maßnahmen sehr leicht wesentlich verbessert werden.

### Beispiel 3

### Lagerung

10 Tabletten nach Beispiel 1 wurden in ein offenes Glasgefäß eingewogen. Die Lagerung erfolgte offen bei RT im Labor bei ca. 50 % rF. Die Gewichtsänderungen wurden über 4 Wochen verfolgt. Es ergab sich keine Gewichtsänderung.

### Beispiel 4

### a) PCR Probenvorbereitung aus humanen Vollblut mit magnetischen Glaspartikeln

### Isolierung der Nukleinsäure

10 mg Glasmagnetpartikel GMP2 wurden je in EppendorfReaktionsgefäßen vorgelegt. Zu je 200 µl aufgetautem Vollblut wurden 40 µl Proteinase K (20 mg/ml, hergestellt aus Lyophilisat) pipettiert und sofort gemischt. Anschließend wurden 200 µl Bindepuffer (6 M Guanidin-HCl, 10 mM Tris-HCl, 10 mM Harnstoff, 30 % Triton X-100, pH 4,4) zugegeben, gemischt und für 10 Minuten bei 70°C inkubiert. Nach Zugabe von 200 µl i-Propanol wurde für 10 Sekunden auf dem Vortex-Mischer gemischt, die Probe für 20 Minuten bei Raumtemperatur inkubiert und abermals für 10 Sekunden wie vor gemischt. Die Magnetseparation erfolgte für wenigstens 30 Sekunden im Magnetpartikelseparator von Boehringer Mannheim (Id.-Nr. 1 641 794). Der Überstand wurde abgenommen und wie weiter unten beschrieben analysiert.

Die Magnetpartikel wurden mit jeweils 500 µl Wasch-Puffer (20 mM NaCl, 10 mM Tris-HCl, pH 7,5 (25°C), 70 % Ethanol) durch 10 Sekunden mischen, 1 Minute Inkubation bei Raumtemperatur und 10 Sekunden mischen gewaschen und mit dem Magnetpartikelseparator an die Gefäßwand gezogen. Der Überstand wurde abgenommen und verworfen. Die Waschprozedur wurde wiederholt bis der Waschüberstand farblos war (insgesamt 5 x Waschen). Nun wurden die Nukleinsäuren 3 x mit jeweils 200 µl auf 70°C vorgewärmten Elutionspuffer (10 mM Tris-HCl, pH 8,5) durch 10 Sekunden mischen, 10 Minuten Inkubation bei Raumtemperatur und 10 Minuten mischen eluiert.

### Aufarbeitung des Überstandes

Der Überstand nach der 1. Bindung an die magnetischen Glaspartikel wurde folgendermaßen auf Gehalt an Nukleinsäuren überprüft: Der Überstand wurde in ein Filter-Tube (Boehringer Mannheim, Id.-Nr. 1744003, z.B. enthalten in High Pure PCR Product Purification Kit) gegeben und für 1 Minute bei 8000 rpm in einer Eppendorf Tischzentrifuge zentrifugiert. Der Durchlauf wird verworfen und das Filter-Tube 2 x mit 500 µl Wasch-Puffer gewaschen (Zentrifugation wie vor). Das Filter-Tube wird kurz trocken zentrifugiert und dann mit 2 x 200 µl auf 70°C vorgewärmten 1 x Elutionspuffer durch erneute Zentrifügation eluiert.

### Analyse der Eluate und des Probenüberstandes

50 µl der Eluate bzw. der über Filter-Tube aufgearbeiteten Überstände wurden mit 10 µl Proben-Puffer versetzt und davon 45 µl in einem 0,8 %igen Agarosegel elektrophoretisch bei 120 V für 90 Minuten aufgetrennt.

Verschiedene Verdünnungen der Eluate bzw. der aufgearbeiteten Überstände wurden bei 260 und 280 nm in einem Uvikon 710 (Kontron) spektroskopisch vermessen.

Zwei 5 µl Aliquots der Eluate wurden als Doppelbestimmung durch Expand™ Long Template PCR (Boehringer Mannheim, Id.-Nr. 1681834) mit spezifischen Primern für das menschliche tPA-Gen (erwartete Produktlänge 15 kb) überprüft.

| Mix I | pro Ansatz | Mix II | pro Ansatz |
|---|---|---|---|
| dNTP, je 100 mM | 1 µl | Expand™ Puffer, 10 x | 5 µl |
| Primer 1, 200 ng/µl | 1 µl | Expand™ Polymerase | 0,75 µl |
| Primer 2, 225 ng/µl | 1 µl | H₂O, _{bidest.} | 19,25 µl |
| H₂O, _{bidest.} | 17 µl | | |
| | 20 µl | | 25 µl |

Mix I wird in ein dünnwandiges PCR-Tube vorgelegt mit 5 µl Eluat versetzt und Mix II zugegeben. Der Ansatz wird kurz gemischt und mit 30 µl Minearalöl überschichtet. Die Ansätze werden in einem Perkin-Elmer Thermocycler 9600 mit folgendem Programm amplifiziert:

| | | |
|---|---|---|
| 2 Minuten | 92°C | |
| | | |
| 10 Sekunden | 92°C | |
| 30 Sekunden | 65°C | 10 Zyklen |
| 12 Minuten | 68°C | |
| | | |
| 10 Sekunden | 92°C | |
| 30 Sekunden | 65°C | 20 Zyklen |
| 12 Minuten + | 68°C | |
| 20 Sekunden pro Zyklus | | |
| 7 Minuten | 68°C | |
| | | |
| anschließend | 7°C | |

Die 50 µl PCR Ansätze wurden mit 10 µl Proben-Puffer versetzt und davon 45 µl in einem 0,8 %igen Agarosegel elektrophoretisch bei 120 V für 90 Minuten aufgetrennt.

### b) Verwendung der erfindungsgemäß tablettierten Pigmente aus Beispiel 2

2 Tabletten nach Beispiel 2 wurden in ein Eppendorf Reaktionsgefäß vorgelegt, 40 ml Proteinase K (20mg/ml) und 200 µl aufgetautes Vollblut wie in Beispiel 4a) zugegeben und sofort 10 sec auf einem Vortex Mischer gemischt. 200 µl 30% Triton X-100 wurden zugegeben und 10 min mit dem Vortex Mischer gemischt. Die weitere Behandlung erfolgte analog zu Beispiel 4a).

### c) Vergleich

Sowohl hinsichtlich der Ausbeute von DNA im ersten Elutionsschritt als auch der Amplifizierbarkeit gab es im Rahmen der üblichen Schwankungen identische Ergebnisse für 4a) und 4b).

## Patentansprüche

1. Reagenzzubereitung zur Bindung von Inhaltsstoffen einer Probe in Form einer Tablette enthaltend
- eine Vielzahl von magnetischen Partikeln mit einer glasartigen Oberfläche, an welche die zu bindenden Inhaltsstoffe im wesentlichen vollständig binden können,
- Tablettierhilfsmittel und
- einem Hilfsstoff, der die Bindung der Inhaltsstoffe erleichtert
**dadurch gekennzeichnet, daß**
der Hilfsstoff ein chaotropes Salz ist.

2. Reagenzzubereitung gemäß Anspruch 1, **dadurch gekennzeichnet, daß** die magnetischen Partikel weniger als 100 µm im Durchmesser betragen.

3. Reagenzzubereitung gemäß Anspruch 1, **dadurch gekennzeichnet, daß** die Tablette schwerer ist als 5 mg.

4. Verwendung einer Reagenzzubereitung gemäß einem der vorangehenden Ansprüche zur Bindung von Nukleinsäuren.

5. Verwendung einer Reagenzzubereitung gemäß einem der vorangehenden Ansprüche zur Reinigung von Nukleinsäuren.

6. Verfahren zur Suspension von magnetischen Partikeln in einer Probe enthaltend die Schritte
- Einbringen einer magnetische Partikel und lösliche Tablettierhilfsstoffe enthaltenden Reagenzzubereitung in Form einer Tablette gemäß einem der Ansprüche 1 bis 3 in die Probe, und
- Bewegung der Tablette in der Probe.

7. Verfahren zum Einbringen von magnetischen Partikeln in eine Probe enthaltend die Schritte
- Zurverfügungstellung eines Spenders enthaltend eine Vielzahl von magnetpartikelhaltigen Tabletten gemäß einem der Ansprüche 1 bis 3, und
- Betätigung des Spenders zum Entlassen einer Tablette aus dem Spender.

## Claims

1. Reagent preparation for binding components of a sample in the form of a tablet containing
- a plurality of magnetic particles having a glass-like surface to which the binding components can essentially completely bind,
- tabletting excipients and
- an auxiliary agent which facilitates the binding of the components
**characterized in that**
the auxiliary agent is a chaotropic salt.

2. Reagent preparation as claimed in claim 1, **characterized in that** the magnetic particles have a diameter of less than 100 µm.

3. Reagent preparation as claimed in claim 1, **characterized in that** the tablet is heavier than 5 mg.

4. Use of a reagent preparation as claimed in one of the previous claims to bind nucleic acids.

5. Use of a reagent preparation as claimed in one of the previous claims to purify nucleic acids.

6. Method for suspending magnetic particles in a sample comprising the steps
- adding a reagent preparation in the form of a tablet containing magnetic particles and soluble tabletting excipients as claimed in one of the claims 1 to 3 to the sample and
- moving the tablet in the sample.

7. Method for introducing magnetic particles into a sample comprising the steps
- providing a dispenser containing a plurality of tablets containing magnetic particles as claimed in one of the claims 1 to 3 and
- activating the dispenser to release a tablet from the dispenser.

## Revendications

1. Préparation de réactifs pour la liaison de constituants d'un échantillon, sous la forme d'un comprimé contenant
- une multitude de particules magnétiques possédant une surface vitreuse à laquelle peuvent se lier de manière essentiellement complète les constituants à lier,
- des adjuvants de transformation en comprimés et
- un adjuvant qui facilite la liaison des constituants,
**caractérisée en ce que** l'adjuvant est un sel chaotrope.

2. Préparation de réactifs selon la revendication 1, **caractérisé en ce que** le diamètre des particules magnétiques est inférieur à 100 µm.

3. Préparation de réactifs selon la revendication 1, **caractérisée en ce que** le comprimé pèse plus de 5 mg.

4. Utilisation d'une préparation de réactifs selon l'une quelconque des revendications précédentes, pour la liaison d'acides nucléiques.

5. Utilisation d'une préparation de réactifs selon l'une quelconque des revendications précédentes, pour la purification d'acides nucléiques.

6. Procédé pour la mise en suspension de particules magnétiques dans un échantillon, comprenant les étapes consistant à :
- introduire dans l'échantillon une préparation de réactifs contenant des particules magnétiques et des adjuvants solubles de transformation en comprimés, sous la forme d'un comprimé selon l'une quelconque des revendications 1 à 3, et
- mouvoir le comprimé dans l'échantillon.

7. Procédé pour l'introduction de particules magnétiques dans un échantillon, comprenant les étapes consistant à :
- mettre à disposition un distributeur contenant une multitude de comprimés contenant des particules magnétiques, selon l'une quelconque des revendications 1 à 3, et
- activer le distributeur pour faire en sorte que celui-ci cède un comprimé.
